# EUROPEAN PATENT SPECIFICATION

(11) **EP 1 624 307 B1**
(45) Date of publication and mention of the grant of the patent: **17.09.2008**
(21) Application number: 05254842.7
(22) Date of filing: 03.08.2005
(51) Int. Cl.: G01N 33/72, G01N 33/543, G01N 33/68

(54) **Assay for detecting glycated or glycosylated haemoglobin (HbA1c)**
Assay zum Nachweis von glykiertem oder glykosyliertem Hämoglobin (HbA1c)
Méthode pour détecter l'hémoglobine glyquée ou glycosylée (HbA1c)

(30) Priority: 03.08.2004 US 598511; 03.08.2004 GB 0417212; 20.04.2005 GB 0507955
(43) Date of publication of application: 08.02.2006
(73) Proprietor: Axis-Shield Diagnostics Limited, Dundee DD2 1XA (GB)
(72) Inventor: Milne, Ken c/o Axis Shield, Dundee DD2 1XA (GB); Lawlor, Margaret c/o Axis-Shield, Dundee, DD2 1XA (GB)
(74) Representative: Main, Malcolm Charles

(56) References cited:
- EP-A- 0 315 866
- EP-A- 1 314 982
- WO-A-98/51435
- WO-A-02/086512
- WO-A1-96/15151
- WO-A1-20/05035092
- US-A- 5 736 349
- GILMAN J G ET AL: "The oxygen-linked zinc-binding site of human haemoglobin." THE BIOCHEMICAL JOURNAL. 1 MAR 1978, vol. 169, no. 3, 1 March 1978 (1978-03-01), pages 625-632, XP002394620 ISSN: 0264-6021
- ARNONE A ET AL: "The binding of zinc to human deoxyhemoglobin and its possible relevance to the anti-sickling effect of zinc." PROGRESS IN CLINICAL AND BIOLOGICAL RESEARCH. 1977, vol. 14, 1977, pages 317-328, XP009070819 ISSN: 0361-7742
- RIFKIND J M ET AL: "Interaction of zinc and hemoglobin: binding of zinc and the oxygen affinity." BIOCHEMISTRY. 4 OCT 1977, vol. 16, no. 20, 4 October 1977 (1977-10-04), pages 4438-4443, XP009070813 ISSN: 0006-2960
- CUNNINGHAM J J ET AL: "Hyperzincuria in individuals with insulin-dependent diabetes mellitus: concurrent zinc status and the effect of high-dose zinc supplementation." METABOLISM: CLINICAL AND EXPERIMENTAL. DEC 1994, vol. 43, no. 12, December 1994 (1994-12), pages 1558-1562, XP002394621 ISSN: 0026-0495
- FU J.Y. ET AL: 'Cloning, expression, purification, and characterization of rat MMP-12' PROTEIN EXPRESSION AND PURIFICATION vol. 21, no. 2, March 2001, UNITED STATES, pages 268 - 274, XP002237748

## Description

### Field of the Invention

The present invention provides improvements in and relating to assay methods for the detection of glycated haemoglobin A1c (HbAlc). Specifically, the present invention provides microparticles having zinc coating for use in the improved binding of proteins exhibiting a zinc binding domain and in particular haemoglobin, such as glycated haemoglobin.

### Background of the Invention

Haemoglobin (Hb) is the protein present in red blood cells which is responsible for the transportation of oxygen. Haemoglobin occurs in several variants, the most common form being haemoglobin A which comprises about 90% of the total haemoglobin found in blood.

A1c is a specific subtype of haemoglobin A. HbAlc is a glycated form of haemoglobin which is formed by the non-enzymatic glycation of the N-terminal valine group of the haemoglobin (Ao) beta chain. In a non-diabetic individual, around 90% of their total haemoglobin is present in a non-glycated form (haemoglobin Ao). Proteins in solution in body fluids are continually subject to glycation processes. Glucose binds slowly to haemoglobin A to form the A1c glycated subtype. The formation of HbAlc reaches a steady state with about 3.0% to 6.5% of haemoglobin being of the A1c subtype. Most diabetic individuals have a higher blood glucose level than non-diabetics. This results in a higher HbAlc level in diabetic patients, with type 1 (juvenile) and type 2 (adult onset) diabetes sufferers having a HbA1c level ranging from about 6% to 15%.

Diabetes Mellitus is a disease associated with poor glycaemic control. Diabetes related complications may be reduced by long term monitoring and tight control of blood glucose levels. In the diabetic patient where blood glucose levels are obviously elevated the HbAlc level can also increase.

The quantification of an individual's HbAlc level can act as an indicator of the average recent blood glucose level which can in turn indicate the possible level of glycation damage to tissues, and thus the likelihood of possible associated diabetic complications if this state remains for the long term.

The HbAlc test is well established as a good index for an individual's glycemic control. The HbAlc test (which is also known as the haemoglobin A1c test, the glycated haemoglobin A1c test, the glycohaemoglobin A1c test or the A1c test) is a laboratory based assay which reveals the average blood glucose level over a period of two to three months. The test measures the number of glucose molecules attached to haemoglobin. As erythrocytes are recycled after their normal lifetime of around 90 to 120 days, the measurement of the attached glucose in a current blood sample allows average blood sugar levels over the previous 2 to 3 months to be determined.

The use of the HbAlc test to monitor longer term glucose levels serves to complement the continuous day to day monitoring of blood glucose levels which can be performed directly by an individual determining their glucose concentration in blood, plasma or urine.

The monitoring of HbAlc levels is currently one of the best ways to monitor diabetes mellitus in order to assess whether the condition is being properly controlled (DCCT clinical study conducted from 1983 to 1993 by the National Institute of Diabetes and Digestive and Kidney Diseases). Specifically, the test allows a physician to ensure that diabetic patients are achieving optimal glycaemic control and further ensures that diabetic patients are properly controlling their glucose levels between physician check ups.

It is recommended that all individuals with type 2 diabetes should have an HbA1c test at least twice a year. In cases where the resulting blood glucose level is too high, a repeat test at more frequent intervals is recommended. This testing results in around 40 million tests being conducted each year in Europe and the US alone.

Regular assessment of HbA1c levels allows an individual to manage their condition, this resulting in the delay or prevention of serious eye, kidney and nerve disease in people with diabetes. There is also an associated improvement in the general health of the individual.

Current methods used to measure HbA1c levels include column chromatography on ion exchange or affinity resins and high performance liquid chromatography (HPLC). However, none of these methods are ideal as methods for measuring HbA1c levels as they suffer from a number of drawbacks such as not being accurate or easily standardised, being expensive and/or involving complicated procedures.

EP-A-1 314 982 describes an agglutination immunoassay and exemplifies haemoglobin A1c derived from a blood sample as being suitable for detection thereby. EP-A-0 315 866 discloses a latex agglutination immunoassay for determining the presence of an analyte in a blood sample. Gilman J. et al. (The Biochemical Journal. 1 Mar 1978 vol 169, no 3, 1 March 1978) discloses that zinc is capable of binding to haemoglobin. Arnone et al. (Progress in clinical and biological research, 1977, p317-328) discloses the presence of a zinc binding site in crystals of deoxyhemoglobin. Rifkind et al. (Biochemistry, 4 October 1977 vol 16, No. 20, pages 4438-4443) discuses the effect of oxygenation of haemoglobin on binding with zinc. Fu et al. (Protein Expression and Purification, vol 21, No. 2, March 2001 (2001-03), pages 268-274) teaches of a method for the purification of proteins using zinc chelate chromatography. International PCT application No. WO 96/15151 teaches of the purification of zinc using immobilised metal affinity chromatography.

There accordingly exists a need for a fast, reliable and easy to use method for the accurate detection of HbA1c levels.

### Summary of the Invention

The present inventors have now surprisingly found that a microparticle coated with zinc exhibits improved binding of haemoglobin and derivatives such as glycated haemoglobin. Thus, these microparticles can be used in methods for the detection of glycated haemoglobin levels.

According to a first aspect of the present invention there is provided an assay method for the determination of the level of glycated haemoglobin within a sample, the method comprising the steps of:
- providing a sample which contains haemoglobin,
- extracting the haemoglobin from the sample and partially denaturing the haemoglobin present,
- exposing the haemoglobin to microparticles coated with zinc molecules in order to allow binding of haemoglobin (Hb) and glycated haemoglobin (HbA1c) to the microparticles, and
- determining the amount of HbAlc in the sample.

In one embodiment of the invention the sample is blood, in particular human anti-coagulated whole blood. In further embodiments the sample may be red blood cell pellets, blood haemolysates or blood extracts such as serum. In a yet further embodiment, the sample may be collected on filter paper, dried and then redissolved.

In one embodiment of the invention the haemoglobin is obtained by haemolysis of the sample in order to release the cell bounded haemoglobin. This step is particularly desirable where the blood sample is whole blood or red blood cells. The blood sample can be haemolysed by methods which are well known to the man skilled in the art, such as adding haemolysis agents such as saponin, sodium dodecyl sulphate (SDS) and/or quarternary ammonium salt.

The invention further provides for haemolysis to be performed using heat.

In one embodiment of the invention partial denaturation of the haemoglobin during the treatment step is performed by detergents. An optimised combination of detergents may be formulated in order to effect the preferred degree of denaturation of the haemoglobin. One aspect of the invention provides for preferred detergents such as cholate, N-octyl-glucoside, N-octyl-thioglucoside, triton X, zwittergent3-14 and bile salts. The importance of denaturing the haemoglobin is accorded to the fact that this results in a conformational change in the haemoglobin molecule. This conformational change facilitates and permits high affinity binding of zinc to the haemoglobin molecule. The occurrence of high affinity binding is particularly preferred in relation to the present invention as it permits the zinc and haemoglobin to remain in an associated bound state during any washing step or steps which may be performed during assay methods. High affinity binding results in a bond between the zinc molecule and haemoglobin molecule which is of a sufficient affinity to withstand washing during an assay procedure. The level of affinity which could be defined as being a high affinity bond can be readily determined by the person skilled in the art through the performance of routine experimentation.

The denaturation of the haemoglobin provides for the formation of a binding site which permits high affinity binding to zinc molecules, while at the same time retaining an epitope to which an antibody or similar binding member can bind in order to indicate the presence of the haemoglobin.

In a preferred embodiment of the invention the sample is first haemolysed before the haemoglobin contained therein is partially denatured.

In one embodiment the step of exposing the haemoglobin to the zinc coated microparticles is performed at a pH of around 7 and preferably at a pH of between pH 6 and 8.

The invention further provides for the microparticle to be a bead such as a latex bead, silica bead, chelating sepharose bead or a magnetic bead. A latex bead is preferred. Carboxyl groups may be provided on the surface of the latex bead in order to facilitate the attachment of zinc molecules. As an alternative, silica beads may be used. Silica beads are however generally heavier than latex beads and accordingly may be less preferred for performing the assays of the present invention, particularly where these assays are automated. Where the microparticle is silica, preferably it has a diameter of 10 to 50 micrometers, more preferably 20 to 30 micrometeres. In one embodiment the silica particle is hollow or substantially hollow in order to reduce its density.

In a further embodiment the microparticle is dextran or polyethylene glycol (PEG). Where the microparticle is dextran, it may preferably form a dextran backbone.

In a preferred embodiment of the invention, a chelator may be bound to the surface of the bead, the chelator allowing zinc molecules to be conjoined thereto. Advantageously, the chelator serves to space the bound zinc molecule away from the surface of the microparticle, when in a bound state.

The chelator may be any suitable zinc binding chelating agent. The invention provides for the chelator to be ethylenediamine tetraacetic acid (EDTA), trisodium calcium diethylenetriamin-pentaacetate (DTPA), disodium calcium cyclohexanediaminetetraacetate (CDTA), sodium calcium edetate, DMSA and DMPS. Other chelating agents include Ethylenediamine (EDA), Diethylenetriamine (DETA) and Aminoethylethanolamine (AEEA) ethyleneamines or any other chelator having zinc-complexing properties similar to those listed.

In one embodiment the zinc coating serves to activate the microparticle. Typically zinc chloride is used to coat the microparticles. Alternatively any suitable zinc salt or elemental form of zinc may be used to bind the surface of the microparticle.

In a further embodiment the microparticle is a chelating bead which is activated by a pre-charged zinc particle.

In one embodiment of the invention, following binding to the microparticles of the present invention, the determination of the amount of HbAlc present may be performed by contacting the HbAlc with signal-generating molecules which selectively bind to HbA1c and thus label it and make it detectable. Suitable signal generating molecules may be, in particular, direct labels, enzyme labels or radiolabels which can bind selectively to glycated haemoglobin, but not to non-glycated haemoglobin. This signal generating molecule may be bound to HbAlc by means of a specific binding partner such as an antibody, preferably a monoclonal antibody but other less preferred binders like boronic acids can also be used. The invention further provides for the signal generating molecule to be conjugated to the antibody.

One such way in which an antibody can be detectably labelled is by conjugating it to an enzyme. This enzyme, in turn, when later exposed to its substrate will catalyse a reaction with the substrate such that a chemical moiety which can be detected results, for example, spectrophotometric or fluorometric means (ELISA system).

Examples of enzymes that can be used as detectable labels are horseradish peroxidase, malate dehydrogenase, staphylococcal nuclease, delta-5-steroid isomerase, yeast alcohol dehydrogenase, alpha-glycerophosphate dehydrogenase, triose phosphate isomerase, alkaline phosphatase, asparaginase, glucose oxidase, beta-galactosidase, ribonuclease, urease, catalase, glucose-6-phosphate dehydrogenase, glucoamylase, and acetylcholine esterase.

For increased sensitivity in the ELISA system, the procedures described can be modified using biotinylated antibody reacting with avidin-peroxidase conjugates. Other methods of detecting biotinylation may further be used. Such methods will be apparent to the man skilled in the art and include detection systems involving streptavidin and systems including peroxidase and alkaline phosphatase detecting systems.

The amount of antigen can also be determined by labelling the antibody with a radioactive isotope. The presence of the radioactive isotope would then be determined by such means as the use of a gamma counter or a scintillation counter. Isotopes which are particularly useful are 3H, 125I, 131I, 32P, 35S, 14C, 51Cr, 36C1, 57Co, 58Co, 59we, 75Se, 111In 99mTc, 67Ga, and Y.

Determination of the antigen is also possible by labelling the antibody with a fluorescent compound. When the fluorescently labelled molecule is exposed to light of the proper wavelength, its presence can then be detected due to fluorescence of the dye. Among the most important fluorescent labelling compounds are fluorescein isothiocyanate, rhodamine, phycoerythrin, phycocyanin, allophycocyanin, o-phthaldehyde, and fluorescamine.

Fluorescence emitting metal atoms such as Eu (europium), and other lanthanides, can also be used. These can be attached to the desired molecule by means of metal chelating groups, such as DTPA or EDTA.

Another way in which the antibody can be detectably labelled is by coupling it to a chemiluminescent compound. The presence of the chemiluminescent-tagged immunoglobulin is then determined by detecting the presence of luminescence that arises during the course of a chemical reaction. Examples of particularly useful chemiluminescent labelling compounds are luminol, isoluminol, aromatic acridinium ester, imidazole, acridinium salt, and oxalate ester.

Likewise, a bioluminescent compound may also be used as a label. Bioluminescence is a special type of chemiluminescence which is found in biological systems wherein a catalytic protein increases the efficiency of the chemiluminescent reaction. The presence of a bioluminescent molecule would be determined by detecting the presence of luminescence. Important bioluminescent compounds for purposes of labelling are luciferin, luciferase, and aequorin.

The use of chemiluminescent substrates for HRP and alkaline phosphatase could also be used.

The measurement of bound haemoglobin could be quantified using UV absorbers in resins such as resorcinol.

In order to ensure that the greatest proportion of glycated haemoglobin in the sample interacts with the signal generating molecules, it is advantageous to use an excess of signal-generating molecules.

In one embodiment all glycated haemoglobin contained in the sample is bound by signal-generating molecules and is thus detectable and distinguishable from non-glycated haemoglobin.

It is important for the method according to this aspect of the invention that the fraction of the signal generating molecules which is not bound to glycated haemoglobin when contacted with the sample is separated from the sample.

A second aspect of the present invention provides an assay method for the *in-vitro* diagnosis and monitoring of diabetes mellitus through the assessment of HbAlc glycated haemoglobin levels, the method comprising the steps of:
- providing a sample obtained from an individual which contains haemoglobin,
- haemolysing the sample to release cell bounded haemoglobin and partially denaturing the haemoglobin,
- capturing the glycated haemoglobin on microparticles coated with zinc,
- exposing the sample to signal generating molecules capable of binding to the glycated haemoglobin present in the sample,
- removing signal generating molecules not bound to glycated haemoglobin, and
- quantifying the amount of bound signal generating molecules to determine the percentage level of HbAlc present in the sample.

In one embodiment of the invention the blood sample is human anti-coagulated whole blood. In a further embodiment the blood sample may be red blood cell pellets, blood haemolysates or blood extracts. The invention further provides for the blood sample to be collected on filter paper, dried and subsequently redissolved.

In one embodiment, haemolysis of the sample to release the cell bound haemoglobin is performed by any method which is well known to the man skilled in the art, such as adding haemolysis agents such as saponin, sodium dodecyl sulphate (SDS) and/or quarternary ammonium salt.

The invention further provides for haemolysis to be performed using heat.

In a further embodiment partial denaturation of the haemoglobin during the treatment step is performed using detergents. Examples of preferred detergents include cholate, N-octyl-glucoside, N-octyl-thioglucoside, triton X, zwittergent3-14 and bile salts.

In a preferred embodiment the sample is first haemolysed before partial denaturation of the haemoglobin occurs. In an alternative embodiment, the partial denaturation of the haemoglobin occurs concurrently with the haemolysis of the sample.

In one embodiment the step of capturing the haemoglobin upon the zinc coated microparticles is performed at a pH of around 7 and preferably at a pH of between pH 6 and 8.

In one embodiement, the method includes the further step of removing cell debris by centrifugation, this step immediately following the haemolysis of the blood sample.

The invention further provides for the microparticle to be a bead such as a latex bead, silica bead, chelating sepharose bead or a magnetic bead. A latex bead is preferred. Carboxyl groups may be provided on the surface of the latex bead in order to facilitate the attachment of zinc molecules. As an alternative, silica beads may be used. Silica beads are however generally heavier than latex beads and accordingly may be less preferred for performing the assays of the present invention, particularly where these assays are automated. Where the microparticle is silica, preferably it has a diameter of 10 to 50 micrometers, more preferably 20 to 30 micrometeres. In one embodiment the silica particle is hollow or substantially hollow in order to reduce its density.

In a further embodiment the microparticle is dextran or polyethylene glycol (PEG). Where the microparticle is dextran, it may preferably form a dextran backbone.

In a preferred embodiment of the invention, a chelator may be bound to the surface of the bead, the chelator allowing zinc molecules to be conjoined thereto. Advantageously, the chelator serves to space the bound zinc molecule away from the surface of the microparticle, when in a bound state.

The chelator may be any suitable zinc binding chelating agent. The invention provides for the chelator to be ethylenediamine tetraacetic acid (EDTA), trisodium calcium diethylenetriamin-pentaacetate (DTPA), disodium calcium cyclohexanediaminetetraacetate (CDTA), sodium calcium edetate, DMSA and DMPS. Other chelating agents include Ethylenediamine (EDA), Diethylenetriamine (DETA) and Aminoethylethanolamine (AEEA) ethyleneamines or any other chelator having zinc-complexing properties similar to those listed.

In one embodiment the zinc coating serves to activate the microparticle. Typically zinc chloride is used to coat the microparticles. Alternatively any suitable zinc salt or elemental form of zinc may be used to bind the surface of the microparticle.

In a further embodiment the microparticle is a chelating bead which is activated by a pre-charged zinc particle.

Analysis for glycated haemoglobin can be carried out on a haemoglobin source, e.g. haemolysed erythrocytes. The determination can be performed by immunoassay such as by competitive or immunometric assay types. Examples of the latter type are radioimmunometric assays (IRMA) and enzyme linked immunosorbent assays (ELISA). In a competitive assay the antigen (i.e. the glycated haemoglobin) is labelled with a detectable label. The sample containing the antigen is incubated with the glycated haemoglobin-specific antibody and the labelled antigen, and after formation of immune complexes, separation and detection, the level of glycated haemoglobin in the sample is determined.

In a preferred embodiment, the percentage level of HbAlc present in the sample is determined through the further steps of:
- capturing the microparticles on a suitable base medium,
- washing the microparticles to remove unbound material,
- labelling the HbAlc with a signal generating molecule being a monoclonal antibody specific for HbAlc, the monoclonal antibody preferably having a label conjugated thereto, and
- determining the percentage of HbAlc present in the sample.

In one embodiment the base medium is a glass fibre matrix.

In another embodiment the monoclonal antibody specific for HbAlc is anti-HbA1c antibody. The anti-HbA1c monoclonal antibody has an epitope specific for the monosaccharide containing N-terminal 8 amino acids of HbA1c.

In a further embodiment the enzyme conjugated to the antibody is an alkaline phosphatase.

In a further embodiment the monoclonal antibody is labelled with an enzyme. In a preferred embodiment the enzyme is alkaline phosphatase, said alkaline phosphatase catalysing the removal of a phosphate group present in a substrate. The enzyme conjoined to the antibody serves to react with components of a substrate introduced about the bound microparticles in order to produce a signal which can be measured to determine the percentage of HbAlc present in a sample.

Where the enzyme conjoined to the antibody is alkaline phosphatase, preferably the substrate is 4-Methylumbelliferyl Phosphate, the alkaline phosphatase labelled conjugate catalysing the removal of the phosphate group from the substrate, yielding the fluorescent product 4-Methylumbelliferyl Phosphate. In such an embodiment, preferably the amount of fluorescence produced is directly linked to the percentage of HbAlc present in the sample, this being determined by reference to a standard response curve.

Conveniently, the amount of fluorescence produced is measured by the MEIA optical assembly. In such an embodiment, fluorescence values were converted to percentages of HbAlc of total haemoglobin by means of a standard curve obtained by plotting the fluorescence at 448nm of samples containing known percentages of HbAlc as determined by HPLC or some other suitable measurement.

### Assay

The invention provides assay systems and screening methods for determining HbAlc glycated haemoglobin levels. As used herein, an "assay system" encompasses all the components required for performing and analysing results of an assay that detects and/or measures a particular event or events.

A variety of assays are available to detect the activity of proteins that have specific binding activity. Exemplary assays use fluorescence polarisation, and laser scanning techniques to measure binding of fluorescently labelled proteins, peptides or other molecules (Lynch, B.A et al. 1999. Anal Biochem 275:62-73; Li HY, 2001, J Cell Biochem 80:293-303; Zuck P et al., Proc Natl Acad Sci USA 1999, 96: 11122-11127).

In another example, binding activity is detected using the scintillation proximity assay (SPA), which uses a biotinylated peptide probe captured on a streptavidin coated SPA bead and radio-labeled partner molecule. The assay specifically detects the radio-labelled protein bound to the peptide probe via scintillant immobilized within the SPA bead (Sonatore LM et al., 1996, Anal Biochem 240:289-297).

In preferred embodiments screening assays are high throughput or ultra high throughput and thus provide automated, cost-effective means of screening.

In one embodiment of the invention the assay can be automated and in a first preferred embodiment would allow between 40 to 60 tests to be performed in an hour. In a second preferred embodiment, the automation would allow between 100 to 160 tests to be performed per hour.

In preferred embodiments of the invention, screening is performed using an automated analyser.
Preferably the automated AxSYM^{TM} system of Abbott Laboratories is used. Techniques used for automated sample analysis and throughput include Abbott IMx^{TM} and microparticle enzyme immunoassay.

The methods of the invention extend to methods of purifying haemoglobin from a sample, for example a blood sample. The haemoglobin need not be glycated.

In a yet further aspect of the present invention there is provided a method of isolating haemoglobin from a biological sample, the method comprising the steps of:
- providing a biological sample from a patient,
- partially denaturing the haemoglobin present in the sample,
- capturing the haemoglobin on microparticles which are pre-coated with zinc chloride, and
- eluting the bound haemoglobin from the zinc coated microparticle.

In one embodiment the method further includes the step of haemolysing the sample and partially denaturing the haemoglobin present in the sample prior to the capturing step.

In one embodiment the biological sample is a blood sample.

In one embodiment, haemoglobin obtained by this method isolates haemoglobin, derivatives and analogues and in particular glycated forms of haemoglobin such as glycated subtype HbA1c.

Zinc coated microparticles can be used in the selective binding of a target protein bearing a zinc binding domain.

Said target proteins are haemoglobin and associated glycated forms, analogues and derivatives.

The microparticle can be a bead such as a latex bead, silica bead, chelating sepharose bead or a magnetic bead. A latex bead is preferred. Carboxyl groups may be provided on the surface of the latex bead in order to facilitate the attachment of zinc molecules. As an alternative, silica beads may be used. Silica beads are however generally heavier than latex beads and accordingly may be less preferred for performing the assays of the present invention, particularly where these assays are automated. Where the microparticle is silica, preferably it has a diameter of 10 to 50 micrometers, more preferably 20 to 30 micrometeres. In one embodiment the silica particle is hollow or substantially hollow in order to reduce its density.

The microparticle can be dextran or polyethylene glycol (PEG). Where the microparticle is dextran, it may preferably form a dextran backbone.

A chelator can be bound to the surface of the bead, the chelator allowing zinc molecules to be conjoined thereto. Advantageously, the chelator serves to move the bound zinc molecule away from the surface of the microparticle, when in a bound state.

The chelator may be any suitable zinc binding chelating agent. Typically the chelator is ethylenediamine tetraacetic acid(EDTA), trisodium calcium diethylenetriamin-pentaacetate (DTPA), disodium calcium cyclohexanediaminetetraacetate (CDTA), sodium calcium edetate, DMSA and DMPS. Other chelating agents include Ethylenediamine (EDA), Diethylenetriamine (DETA) and Aminoethylethanolamine (AEEA) ethyleneamines or any other chelator having zinc-complexing properties similar to those listed.

Where the microparticule is Dextran, preferably the chelator is EDTA.

A kit for the performance of any one of the assay methods of the invention is provided, said kit comprising microparticles together with instructions and protocols for the performance of the assay method(s).

Advantageously, the kit provides instructions for the performance of the methods using the AxSYM automated assay system commercially available and provided by Abbott Laboratories (US).

Microparticle coated with zinc molecules are provided.

The microparticle can be a bead such as a polystyrene bead, silica bead, chelating sepharose bead or a magnetic bead. The zinc can be directly coupled on the surface of the microparticle or deposited on the surface by vacuum deposition.

Where the microparticle is silica, preferably it has a diameter of 10 to 50 micrometers, more preferably 20 to 30 micrometeres. In one embodiment the silica particle is hollow or substantially hollow in order to reduce its density.

The microparticle can be dextran or polyethylene glycol (PEG).

A chelator can be bound to the surface of the bead, the chelator allowing zinc molecules to be conjoined thereto. Advantageously, the chelator serves to space the bound zinc molecule away from the surface of the microparticle.

The chelator may be any suitable zinc binding chelating agent. For example, the chelator may be ethylenediamine tetraacetic acid (EDTA), trisodium calcium diethylenetriamin-pentaacetate (DTPA), disodium calcium cyclohexanediaminetetraacetate (CDTA), sodium calcium edetate, DMSA or DMPS. Other chelating agents include Ethylenediamine (EDA), Diethylenetriamine (DETA) and Aminoethylethanolamine (AEEA) ethyleneamines or any other chelator having zinc-complexing properties similar to those listed

Where the microparticle is dextran, the preferably the chelator is EDTA.

The zinc coating can serve to activate the microparticle. Typically zinc chloride is used to coat the microparticles, however any suitable soluble zinc salt or elemental form of zinc may be used to coat the surface of the microparticle. Without wishing to be bound by theory, it is predicted that the microparticle will not be completely covered by the bound zinc molecule. However there will be a high level of zinc bound molecules in relation to the available surface area.

The microparticle can be a chelating bead which is activated by a pre-charged magnetic zinc particle.

There is provided a compound for use in the targeted binding of proteins bearing a zinc binding site, the compound comprising a microparticle coated with zinc molecules.

The microparticle can be a bead such as a polystyrene bead, a silica bead, a chelating sepharose bead or a magnetic bead.

Where the microparticle is silica, preferably it has a diameter of 10 to 50 micrometers, more preferably 20 to 30 micrometeres. In one embodiment the silica particle is hollow or substantially hollow in order to reduce its density.

A chelator can be bound to the surface of the bead, the chelator allowing zinc molecules to be conjoined thereto. Advantageously, the chelator serves to space the bound zinc molecule away from the surface of the microparticle, when in a bound state.

The chelator may be any suitable zinc binding chelating agent. Typically the chelator is ethylenediamine tetraacetic acid(EDTA), trisodium calcium diethylenetriamin-pentaacetate (DTPA), disodium calcium cyclohexanediaminetetraacetate (CDTA), sodium calcium edetate, DMSA or DMPS. Other chelating agents include Ethylenediamine (EDA), Diethylenetriamine (DETA) and Aminoethylethanolamine (AEEA) ethyleneamines or any other chelator having zinc-complexing properties similar to those listed.

There is provided a compound for use in the improved binding of glycated haemoglobin, the compound comprising a microparticle coated with zinc.

The microparticle may be a bead such as a polystyrene bead, a silica bead, a chelating sepharose bead or a magnetic bead.

A chelator is bound to the surface of the bead, the chelator allowing zinc molecules to be conjoined thereto. Advantageously, the chelator serves to move the bound zinc molecule away from the surface of the microparticle, when in a bound state.

The chelator may be any suitable zinc binding chelating agent. Typically the chelator is ethylenediamine tetraacetic acid(EDTA), trisodium calcium diethylenetriamin-pentaacetate (DTPA), disodium calcium cyclohexanediaminetetraacetate (CDTA), sodium calcium edetate, DMSA or DMPS. Other chelating agents include Ethylenediamine (EDA), Diethylenetriamine (DETA) and Aminoethylethanolamine (AEEA) ethyleneamines or any other chelator having zinc-complexing properties similar to those listed

The zinc coating serves to activate the microparticle. Typically zinc chloride is used to coat the microparticles. Alternatively any suitable zinc salt or elemental form of zinc may be used to bind the surface of the microparticle.

The microparticle can be a chelating bead which is activated by a pre-charged zinc particle.

Advantageously, the inventors have found that the zinc bound microparticle has improved properties in the binding of haemoglobin, its related derivatives and glycated forms, due to the advantageous surfactant properties provided by the compound. Without wishing to be bound by theory, it is believed that the improved surfactant properties exhibited by the compound allow haemoglobin to be more efficiently extracted from a membrane bounded state, through conferring improved washing conditions and allowing fast and efficient rupture of the cell membrane to occur and thereafter, efficient haemoglobin binding.

Unless otherwise defined, all technical and scientific terms used herein have the meaning commonly understood by a person who is skilled in the art in the field of the present invention.

### Detailed description of the Invention

The present invention will now be described with reference to the following examples which are provided for the purpose of illustration and are not intended to be construed as being limiting on the present invention, and further, with reference to the figures wherein:

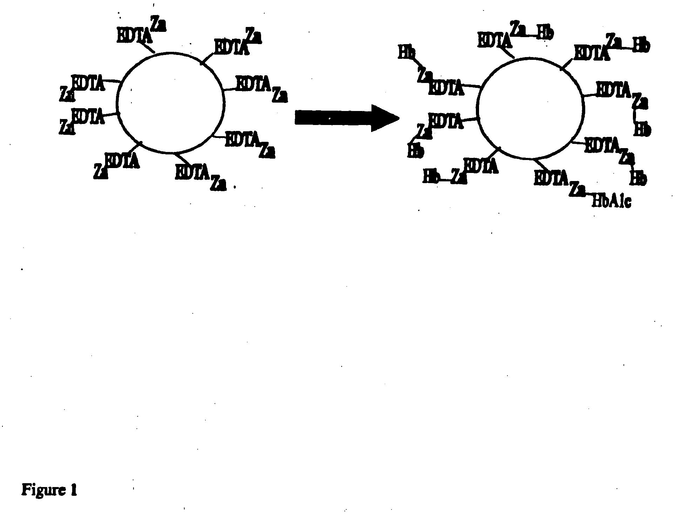

Figure 1 shows an illustrative example of the capture of haemoglobin (Hb) and glycated haemoglobin subtype HbAlc and zinc coated microparticles of the invention, the zinc being bound to the microparticle by an EDTA intermediate, and

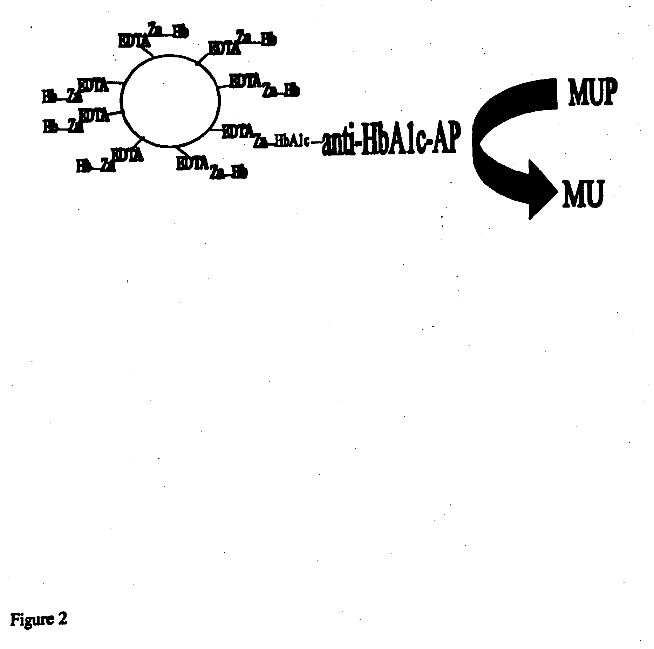

Figure 2 shows a preferred method for the detection of a bound target protein, in this case glycated haemoglobin subtype HbAlc, following the binding of the microparticle.

### EXAMPLES AND COMPARATIVE EXAMPLES

### Example 1

### AxSYM automated assay conditions

Manually lyse blood with lysis buffer supplemented with 0.5% TX-100 and 0.8mM zwittergent 3-14.

Pre-wash tab with MEIA + 5% BSA.
Mix lysed blood sample with equal volume zinc coated EDTA silica microparticles in 30% sucrose and incubate for 5 minutes.
Transfer to tab and wash with MEIA.
Add 1µg/ml αHbA1c in blocking buffer 17 and incubate for 5 minutes.
Wash with MEIA.
Add α-mouse-alkaline phosphatase diluted 1 in 3000 with HEPES pH7.
Wash with MEIA.
Add MUP and read.

**Table 1**

| **Sample** | **Blank** | **Rate** | **Ratio** |
|---|---|---|---|
| 1mg/ml Hb | 175 | 312 | 1:1.8 |
| RBC (5% HbA1c) | 211 | 371 | 1:1.8 |
| RBC (9% HbA1c) | 211 | 531 | 1:2.5 |

Assay detects presence of HbAlc and can discriminate between 5% and 9% HbAlc levels.

### Example 2

### IMx automated assay conditions

Pre-wash tab with MEIA + 5% BSA.
Mix sample and 0.1% zinc coated EDTA silica microparticles in 30% sucrose (1:1).
Apply to tab and wash with MEIA (2 x 50µl).
Add 1µg/ml αHbA1c in HEPES and incubate for 5min.
Wash with MEIA (2 x 50µl).
Add α-mouse-alkaline phosphatase conjugate (diluted 1 in 3000 with HEPES) and incubate for 5min.
Wash with MEIA (2 x 50µl).
Add MUP and read.

**Table 2**

| **Sample** | **Rate** |
|---|---|
| Particles (blank) | 97 |
| HbAlc (0.25 mg) | 330 |
| Hb (0.125 mg)+ HbAlc (0.125 mg) | 175 |
| Hb (0.2 mg)+ HbA1c (0.05 mg) | 132 |
| Hb (0.25mg) | 121 |

Increasing concentrations of HbAlc result in higher rates measured.

### Example 3

### 96 well vacuum manifold assay

### Capturing Hb using Zn microparticles

Silica EDTA and polystyrene NTA microparticles were coated with ZnCl₂ and then incubated at 0.5% solids with Hb (0.5mg/ml) or (0.2mg/ml) for 10 min.
Binding was assessed by measuring the unbound material using OD 405nm. The readings for 0.5mg/ml and 0.2mg/ml fall within the linear range for Hb measurement at 405 nm.

**Table 3: Binding capacity of zinc coated silica EDTA and polystyrene NTA microparticles for haemoglobin.**

| | Measured unbound 0.5mg/ml Hb | | Measured unbound 0.1mg/ml Hb | |
|---|---|---|---|---|
| | Average OD 405nm (- background) | Bound (%) | Average OD 405nm (- background) | Bound (%) |
| polystyrene NTA | 0.249 | 70 | 0.024 | 84 |
| | | | | |
| Silica EDTA 1.5µm (1st batch) | 0.118 | 86 | 0 | 100 |
| | | | | |
| Silica EDTA 1.0µm | 0.093 | 89 | 0 | 100 |
| | | | | |
| Silica EDTA 1.5µm (2nd batch) | 0.111 | 87 | 0 | 100 |
| | | | | |
| Silica EDTA 3.0µm | 0.273 | 67 | 0.037 | 75 |
| | | | | |
| Input Hb 0.5mg/ml | 0.828 | | | |
| Input Hb0.1mg/ml | 0.146 | | | |

This experiment shows that the silica EDTA beads of 1-1.5µm display the highest binding capacity (over 85% for 0.5mg/ml Hb and 100% for 0.1mg/ml Hb) with the NTA and EDTA 3µm showing less binding of Hb (over 65% for 0.5mg/ml Hb and 75% for 0.1mg/ml Hb).

### Detection of captured HbA1c.

The silica EDTA and polystyrene NTA microparticles were coated with zinc and incubated at 0.5% solids with 0.1mg/ml Hb spiked with 0, 1, 5 and 10% HbA1c. Particles were probed with α-HbA1c antibodies (10ug/ml) and α-mouse-alkaline phosphatase (diluted 1:3000) After incubation with alkaline phosphatase substrate the reaction was stopped and OD 550nm was read.

**Table 4: Silica EDTA and polystyrene NTA microparticles.**

| | PBS (Blank) | 0.1mg/ml Hb | Hb+10%HbA1c |
|---|---|---|---|
| polystyrene NTA | 0.204 | 0.273 | 0.262 |
| | | | |
| Silica EDTA 1.5µm (1st batch) | 0.207 | 0.323 | 0.412 |
| | | | |
| Silica EDTA 1.0µm | 0.298 | 0.44 | 0.49 |
| | | | |
| Silica EDTA 1.5µm (2nd batch) | 0.199 | 0.383 | 0.412 |
| | | | |
| Silica EDTA 3.0µm | 0.109 | 0.245 | 0.242 |

The silica EDTA 1-1.5µm microparticles gave the best result with an increase in signal with addition of 10% HbA1c.

### Example 4

### Use of different types of zinc coated microparticles

### Silica EDTA microparticles

Microparticles were coated with zinc and resuspended in 40% sucrose prior to analysis by IMx (method described in Example 2).

**Table 5**

| **Sample** | **Blank** | **Rate** |
|---|---|---|
| RBC (5% HbA1c) | 244 | 677 |
| RBC (9% HbA1c) | 244 | 746 |

The silica particles are able to bind HbAlc and successfully discriminate samples with 5 and 9% HbA1c.

### Polystyrene EDTA microparticles

Microparticles were coated with zinc and resuspended in 30% sucrose prior to analysis by AxSYM (method described in example 1). The αHbA1c (2µg/ml) was incubated for 5min followed by α-mouse-alkaline phosphatase (1:3000) for 2min.

**Table 6**

| **Sample** | **Blank** | **Rate** |
|---|---|---|
| RBC (5% HbA1c) | 112 | 230 |
| RBC (9% HbA1c) | 162 | 494 |

The polystyrene EDTA microparticles are able to bind HbAlc and successfully discriminate samples with 5 and 9% HbA1c.

### Polystyrene NTA microparticles

Microparticles were coated with zinc prior to analysis by vacuum manifold (method described in Example 3).

**Table 7**

| | PBS (Blank) | 0.1mg/ml Hb | Hb+10%HbA1c |
|---|---|---|---|
| polystyrene NTA | 0.204 | 0.273 | 0.262 |

The polystyrene NTA microparticles are able to bind Hb but shows very low or almost no discrimination of HbAlc after spiking 10% HbAlc in sample.

### Magnetic Zn microparticles

Microparticles were analysis by vacuum manifold for binding capabilities (method described in Example 3).

**Table 8**

| | Average OD 405nm (-background) | Bound (%) |
|---|---|---|
| Measured unbound 1.0mg/ml Hb | 0.105 | 85 |
| Measured unbound 0.5mg/ml Hb | 0.051 | 93 |
| Blank (HEPES buffer) | 0.048 | - |
| | | |
| 0.5mg/ml Hb | 0.695 | |

Magnetic Zn microparticles successfully bind Hb.

The bound Hb was analysed for HbAlc using analysis by vacuum manifold (method described in Example 3).

**Table 9**

| | Average OD 550nm |
|---|---|
| 0.1mg/ml Hb | 0.384 |
| Blank (HEPES buffer) | 0.194 |

Magnetic Zn microparticles can be used to detect bound HbA1c.

### Chelating sepharose

Chelating sepharose was coated with zinc and incubated with lysed blood sample using analysis by vacuum manifold for binding capabilities (method described in Example 3).

**Table 10**

| **Sample** | **Unbound Hb (OD405nm)** | **Bound Hb (OD405nm after EDTA elution)** |
|---|---|---|
| Lysed RBC | 0.265 | 2.608 |

Most of the haemoglobin bound to the chelating sepharose.

### Example 5

### Use of different metal ions

### Chelating sepharose

Chelating sepharose was coated with various metal ions and incubated with lysed blood sample using analysis by vacuum manifold for binding capabilities (method described in Example 3).

**Table 11**

| **Metal ion** | **Unbound Hb (OD405nm)** | **Bound Hb (OD405nm after EDTA elution)** |
|---|---|---|
| Zinc chloride | 0.265 | 2.608 |
| Nickel sulphate | 1.953 | 2.165 |
| Copper sulphate | 1.078 | 0.359 |
| Iron chloride | 3.667 | 0.290 |
| Non coated chelating sepharose | 2.361 | 0.695 |

Zinc chloride bound the greatest quantity of haemoglobin.

### Silica EDTA microparticles

Silica EDTA microparticles were coated with various metal ions and analysed by IMx (method described in Example 2).

**Table 12**

| **Metal ion** | **Rate (- blank)** |
|---|---|
| Zinc chloride | 443 |
| Iron chloride | 122 |
| Gold chloride | 91 |

Zinc chloride bound the most HbAlc that could be detected by IMx.

### Example 6

### Use of detergent to lyse RBC and still allow binding and detection of HbAlc on zinc coated microparticles

### Experiment 1

Blood samples were lysed with buffer containing 0.5% TX100 + 0.8mM zwittergent3-14 and analysed by AxSYM (method described in Example 1).

**Table 13**

| **Sample** | **Rate (- Blank)** |
|---|---|
| Normal (5% HbA1c) | 376 |
| High (9% HbA1c) | 568 |

### Experiment 2

Blood samples were lysed with buffer containing 0.5% TX100 + 80mM cholate and analysed by AxSYM (method described in Example 1).

**Table 14**

| **Sample** | **Rate (- Blank)** |
|---|---|
| Normal (5% HbA1c) | 20 |
| High (9% HbA1c) | 465 |

### Experiment 3

Blood samples (normal, 5% HbA1c) were lysed with buffer containing 0.5% TX100 + various detergents and analysed by IMX (method described in Example 2). The HbAlc was detected with αHbA1c-alkaline phosphatase (1µg/ml) in blocking buffer 17.

**Table 15**

| **Sample** | **Rate (- Blank)** |
|---|---|
| 80mM cholate | 840 |
| 250mM N-octyl-glucoside | 807 |
| 90mM N-octyl-thioglucoside | 395 |

Use of various detergents to lyse red blood cell membranes (RBC) does not affect the subsequent binding and detection of HbAlc bound to zinc coated EDTA microparticles.

### Example 7

### Capture of HbAlc on various sized silica microparticles and use of anti-HbA1c-alkaline phosphatase conjugate for detection

Blood samples were lysed with lysis buffer containing 80mM cholate and analysed by IMX (method described in example 2, only one incubation step with conjugate). Samples were incubated with silica microparticles of various sizes (1.5, 0.5 and 0.3µm) at 0.05% solids. The HbAlc was detected with αHbA1c-alkaline phosphatase (1µg/ml) in Hepes pH 7.0, Sodium Azide 0.2%, 2% PEG 1000 and 1.2% StabilGuard conjugate diluent.

**Table 16**

| **Particles (um)** | **Blank** | **Rate** | **Rate- Blank** | **Rate:Blank Ratio** |
|---|---|---|---|---|
| 1.5 | 93 | 1009 | 916 | 11:1 |
| 0.5 | 93 | 422 | 329 | 5:1 |
| 0.3 | 93 | 3329 | 3236 | 36:1 |

### Example 8

### Optimisation of conditions and reagents for performance of automated glycated haemoglobin quantification using the AXSYM automated assay

The optimisation of the conditions for performance of the assays of the present invention has resulted in the following protocol and buffers being identified as preferable for the performance of the present invention using automated analysis on the AXSYM automated assay machine (Abbott Laboratories, US).

### Performance of Assay Methodology

5µl of whole blood sample (EDTA, FLUOX, citrate, heparin) was added to 95µl of RBC lysis buffer (1/20 dilution). The Lysed sample was further diluted with 100µl of sample diluent (final sample dilution of 1/40).

Tab blocker (95µl) was put on the AXSYM tab of the matrix cell with a 20 second delay. The diluted lysed sample (95µl) was transferred to the tab and the tab was washed with 100µl of AxSYN® Solution 3 with a 10 second delay. Conjugate (90µl) was added to the tab and incubated for 300 seconds. The tab was then washed with 2 x 100µl of AxSYM® Solution 3 with a 10 second delay between each pulse and 1 x 50µl of MUP was put on the tab.

The fluorescent product was then read 8 times, with each read taking 506.88msec and 0.6 seconds between each read. The gain was set at 20. Time to first result was 13.7 with a throughput of 46 tests per hour.

The buffers preferred for use in the methods of this example are formulated as follows:

### (i) Working Strength Conjugate

DAKO mouse anti-Hb(A,C,S)1c and anti-HbA1c have been conjugated in-house with alkaline phosphatase using convential maleimide conjugation procedures. They were evaluated and anti-Hb(A,C,S)1c alkaline phosphatase conjugate performed best. The conjugate is used at 0.25µg/ml concentration in conjugate diluent (buffer formulation is 0.05M HEPES, 6% Stabilguard, 2% polyethylene 1000, 0.5% Triton-X100, 1% StabilzymeAP, 0.09% sodium azide, pH 7).

### (ii) RBC lysis buffer

Best performance was achieved using 0.2M HEPES, 0.055M Glycine, 0.05M magnesium chloride, 0.4M sodium choride, 1% Triton-X100, 0.8mM zwittergent 3-14, 0.09% sodium azide, pH 8.

### (iii) Tab blocker/Sample diluent

Variation of AxSYM® matrix cells is minimised and Hb binding is optimal when a tab blocker and sample diluent is used. Currently the two preferred buffers are buffer 3 (0.05M Tris, 20% sucrose, 0.5M sodium chloride, 0.1% fish skin gelatin, 10% Stabilguard, 1mM zinc chloride, 0.09% sodium azide, pH 8) and buffer 7 (0.05M Tris, 20% sucrose, 2M sodium chloride, 0.1% fish skin gelatin, 10% Stabilguard, 5% polyethylene glycol 8000, 0.1% casein hydrolysate, 1mM zinc chloride, 0.09% sodium azide, pH 8).

Various modifications and variations to the described embodiments of the inventions will be apparent to those skilled in the art without departing from the scope of the invention. Although the invention has been described in connection with specific preferred embodiments, it should be understood that the invention as claimed should not be unduly limited to such specific embodiments. Indeed, various modifications of the described modes of carrying out the invention which are obvious to those skilled in the art are intended to be covered by the present invention.

## Claims

1. An assay method for determining the level of glycated haemoglobin within a sample, the method including the steps of:
- denaturing the haemoglobin by means of a conformational change which causes exposure of a high affinity zinc binding site,
- capturing the haemoglobin molecules using zinc molecules coupled to a support medium, and
- determining the amount of glycated haemoglobin bound by the coupled zinc molecules.

2. An assay method as claimed in claim 1 wherein denaturation is caused by detergents.

3. An assay method as claimed in claim 2 wherein the detergents are one or more of the detergents selected from the group consisting of cholate, N-octyl-glucoside, N-octyl-thioglucoside, triton X, zwittergent3-14 and bile salts.

4. An assay method as claimed in any one of claims 1 to 3 wherein capture of the haemoglobin by the coupled zinc molecules is performed in the presence of the denaturation detergents.

5. An assay method as claimed in any one of claims 1 to 3 wherein the zinc molecule is coupled to a microparticle.

6. An assay method as claimed in claim 5 wherein the microparticle is a bead selected from the group consisting of a latex bead, a polystyrene bead, a silica bead, a chelating sepharose bead or a magnetic bead.

7. An assay method as claimed in claim 5 or claim 6 wherein the zinc coupled to the microparticle is provided by zinc chloride.

8. An assay method as claimed in claim 5 wherein the microparticle is dextran or polyethylene glycol (PEG).

9. An assay method as claimed in any preceding claim wherein a chelator facilitates the coupling of the zinc molecules to the support medium.

10. An assay method as claimed in claim 9 wherein the chelator is a zinc binding chelating agent.

11. An assay method as claimed in claim 9 wherein the chelator is selected from the group consisting of ethylenediamine tetraacetic acid (EDTA), trisodium calcium diethylenetriamin-pentaacetate (DTPA), disodium calcium cyclohexanediaminetetraacetate (CDTA), sodium calcium edetate, DMSA, DMPS, Ethylenediamine (EDA), Diethylenetriamine (DETA), and Aminoethylethanolamine (AEEA) ethyleneamines.

12. An assay method as claimed in any preceding claim wherein the haemoglobin containing sample is whole blood, red blood cells, blood haemolysates or blood extracts.

13. An assay method as claimed in claim 12 wherein the haemoglobin containing sample is whole blood cells which are lysed prior to denaturation.

14. An assay method as claimed in claim 12 wherein the red blood cells are lysed using the detergents which also cause denaturation of the haemoglobin.

15. An assay method as claimed in claim 12 wherein the red blood cells are lysed using heat.

16. An assay method for determining the level of glycated haemoglobin within a sample, the method including the steps of:
- extracting the haemoglobin from a blood sample and partially denaturing the haemoglobin present,
- exposing the haemoglobin to microparticles coated with zinc molecules in order to allow binding of haemoglobin and glycated haemoglobin to the microparticles, and
- determining the amount of HbA1c bound to the microparticles.

17. The assay method of claim 16, wherein haemoglobin is extracted from the sample by haemolysis of the sample to release the cell bounded haemoglobin.

18. The assay method of claim 16, wherein the partial denaturation of the haemoglobin during the treatment step is performed by detergents.

19. The assay method of claim 18, wherein the detergents are one or more of the detergents selected from the group consisting of cholate, N-octyl-glucoside, N-octyl-thioglucoside, triton X, zwittergent3-14 and bile salts.

20. The assay method of claim 16, wherein the step of exposing the haemoglobin to the zinc coated microparticles is performed at a pH of around 7.

21. The assay method of claim 16, wherein the determination of the amount of HbA1c present is performed by contacting the HbA1c with signal-generating molecules which selectively bind to HbA1c and thus label it and make it detectable.

22. An assay method for the *in-vitro* diagnosis and monitoring of diabetes mellitus through the assessment of HbA1c glycated haemoglobin levels, the method including the steps of:
- haemolysing a blood sample to release cell bounded haemoglobin and partially denaturing the haemoglobin,
- capturing the glycated haemoglobin on microparticles coated with zinc,
- exposing the captured glycated haemoglobin on the microparticles to signal generating molecules capable of binding to the glycated haemoglobin,
- removing signal generating molecules not bound to glycated haemoglobin, and
- quantifying the amount of bound signal generating molecules to determine the percentage level of HbA1c present in the sample.

23. The assay method of claim 22, wherein the percentage level of HbA1c present in the sample is determined through the further steps of:
- capturing the microparticles on a suitable base medium,
- washing the microparticles to remove unbound material,
- labelling the HbA1c with a signal generating molecule being a monoclonal antibody specific for HbA1c, the monoclonal antibody having a label conjugated thereto, and
- determining the percentage of HbA1c present in the sample.

24. A method of isolating haemoglobin from a biological sample, the method including the steps of:
- partially denaturing the haemoglobin present in the sample,
- capturing the haemoglobin from the sample on microparticles which are coated with zinc, and
- eluting the microparticle-bound haemoglobin from the zinc coated microparticle.

25. The method of claim 24, wherein the biological sample is blood.

## Patentansprüche

1. Ein Assay-Verfahren zum Bestimmen des Grads an glykiertem Hämoglobin innerhalb einer Probe, wobei das Verfahren die folgenden Schritte umfasst:
- Denaturieren des Hämoglobins durch eine Konformationsänderung, was die Freilegung einer Zinkbindungsstelle mit hoher Affinität bewirkt,
- Einfangen der Hämoglobinmoleküle unter Verwendung von Zinkmolekülen, die an ein Trägermedium gekoppelt sind, und
- Bestimmen der Menge an glykiertem Hämoglobin, das durch die gekoppelten Zinkmoleküle gebunden wird.

2. Assay-Verfahren gemäß Anspruch 1, wobei Denaturierung durch Detergenzien bewirkt wird.

3. Assay-Verfahren gemäß Anspruch 2, wobei die Detergenzien eines oder mehrere der Detergenzien sind, die aus der Gruppe ausgewählt sind, die aus Cholat, N-Octyl-Glucosid, N-Octyl-Thioglucosid, Triton-X, Zwittergent 3-14 und Gallensalzen besteht.

4. Assay-Verfahren gemäß einem der Ansprüche 1 bis 3, wobei das Einfangen des Hämoglobins durch die gekoppelten Zinkmoleküle in der Anwesenheit der Denaturierungsdetergenzien durchgeführt wird.

5. Assay-Verfahren gemäß einem der Ansprüche 1 bis 3, wobei das Zinkmolekül an ein Mikropartikel gekoppelt ist.

6. Assay-Verfahren gemäß Anspruch 5, wobei das Mikropartikel eine Perle ist, die aus der Gruppe ausgewählt ist, die aus einer Latexperle, einer Polystyrolperle, einer Siliciumdioxidperle, einer chelatbildenden Sepharoseperle oder einer magnetischen Perle besteht.

7. Assay-Verfahren gemäß Anspruch 5 oder Anspruch 6, wobei das an das Mikropartikel gekoppelte Zink durch Zinkchlorid bereitgestellt wird.

8. Assay-Verfahren gemäß Anspruch 5, wobei das Mikropartikel Dextran oder Polyethylenglykol (PEG) ist.

9. Assay-Verfahren gemäß einem der vorhergehenden Ansprüche, wobei ein Chelator das Koppeln der Zinkmoleküle an das Trägermedium erleichtert.

10. Assay-Verfahren gemäß Anspruch 9, wobei der Chelator ein zinkbindendes chelatbildendes Mittel ist.

11. Assay-Verfahren gemäß Anspruch 9, wobei der Chelator aus der Gruppe ausgewählt ist, die aus Ethylendiamintetraessigsäure (EDTE), Trinatriumcalciumdiethylentriaminpentaacetat (DTPA), Dinatriumcalciumcyclohexandiamintetraacetat (CDTA), Natriumcalciumedetat, DMSA, DMPS, Ethylendiamin (EDA), Diethylentriamin (DETA) und Aminoethylethanolamin(AEEA)-Ethylenaminen besteht.

12. Assay-Verfahren gemäß einem der vorhergehenden Ansprüche, wobei die Hämoglobin enthaltende Probe aus Vollblut, roten Blutzellen, Bluthämolysaten oder Blutextrakten besteht.

13. Assay-Verfahren gemäß Anspruch 12, wobei die Hämoglobin enthaltende Probe aus Vollblutzellen besteht, die vor der Denaturierung lysiert werden.

14. Assay-Verfahren gemäß Anspruch 12, wobei die roten Blutzellen unter Verwendung der Detergenzien lysiert werden, die ebenfalls die Denaturierung des Hämoglobins bewirken.

15. Assay-Verfahren gemäß Anspruch 12, wobei die roten Blutzellen unter Verwendung von Wärme lysiert werden.

16. Assay-Verfahren zum Bestimmen des Grads an glykiertem Hämoglobin innerhalb einer Probe, wobei das Verfahren die folgenden Schritte umfasst:
- Extrahieren des Hämoglobins aus einer Blutprobe und teilweise Denaturieren des vorhandenen Hämoglobins,
- Freilegen des Hämoglobins gegenüber mit Zinkmolekülen beschichteten Mikropartikeln, um das Binden von Hämoglobin und glykiertem Hämoglobin an die Mikropartikel zu ermöglichen, und
- Bestimmen der Menge des an die Mikropartikel gebundenen HbA1c.

17. Assay-Verfahren gemäß Anspruch 16, wobei Hämoglobin aus der Probe durch Hämolyse der Probe extrahiert wird, um das an die Zelle gebundene Hämoglobin freizusetzen.

18. Assay-Verfahren gemäß Anspruch 16, wobei die Teil-Denaturierung des Hämoglobins während des Behandlungsschritts durch Detergenzien durchgeführt wird.

19. Assay-Verfahren gemäß Anspruch 18, wobei die Detergenzien eines oder mehrere der Detergenzien sind, die aus der Gruppe ausgewählt sind, die aus Cholat, N-Octyl-Glucosid, N-Octyl-Thioglucosid, Triton-X, Zwittergent 3-14 und Gallensalzen besteht.

20. Assay-Verfahren gemäß Anspruch 16, wobei der Schritt des Freilegens des Hämoglobins gegenüber den mit Zink beschichteten Mikropartikeln bei einem pH-Wert von etwa 7 durchgeführt wird.

21. Assay-Verfahren gemäß Anspruch 16, wobei die Bestimmung der Menge an vorhandenem HbA1c durch das Kontaktieren des HbA1c mit Signal erzeugenden Molekülen, die sich selektiv an HbA1c binden und es folglich kennzeichnen und nachweisbar machen, durchgeführt wird.

22. Ein Assay-Verfahren für die *In vitro*-Diagnose und das Überwachen von Diabetes mellitus durch die Bewertung von durch HbA1c glykierten Hämoglobin-Graden, wobei das Verfahren die folgenden Schritte umfasst:
- Hämolysieren einer Blutprobe, um an die Zelle gebundenes Hämoglobin freizusetzen und das Hämoglobin teilweise zu denaturieren,
- Einfangen des glykierten Hämoglobins auf mit Zink beschichteten Mikropartikeln,
- Freilegen des eingefangenen glykierten Hämoglobins auf den Mikropartikeln gegenüber Signal erzeugenden Molekülen, die dazu fähig sind, sich an das glykierte Hämoglobin zu binden,
- Entfernen von Signal erzeugenden Molekülen, die nicht an glykiertes Hämoglobin gebunden sind, und
- Quantifizieren der Menge an gebundenen Signal erzeugenden Molekülen, um den Grad in Prozent von in der Probe vorhandenem HbA1c zu bestimmen.

23. Assay-Verfahren gemäß Anspruch 22, wobei der Grad in Prozent an in der Probe vorhandenem HbA1c durch die folgenden weiteren Schritte bestimmt wird:
- Einfangen der Mikropartikel auf einem geeigneten Basismedium,
- Waschen der Mikropartikel, um nicht gebundenes Material zu entfernen,
- Kennzeichnen des HbA1c mit einem Signal erzeugenden Molekül, das ein für HbA1c spezifischer monoklonaler Antikörper ist, wobei der monoklonale Antikörper eine dazu konjugierte Kennzeichnung aufweist, und
- Bestimmen des Prozentsatzes an in der Probe vorhandenem HbA1c.

24. Ein Verfahren zum Isolieren von Hämoglobin aus einer biologischen Probe, wobei das Verfahren die folgenden Schritte umfasst:
- teilweise Denaturieren des in der Probe vorhandenen Hämoglobins,
- Einfangen des Hämoglobins aus der Probe auf Mikropartikeln, die mit Zink beschichtet sind, und
- Eluieren des an das Mikropartikel gebundenen Hämoglobins aus dem mit Zink beschichteten Mikropartikel.

25. Verfahren gemäß Anspruch 24, wobei die biologische Probe Blut ist.

## Revendications

1. Une méthode d'analyse destinée à déterminer le niveau d'hémoglobine glyquée au sein d'un échantillon, la méthode incluant les étapes consistant à :
- dénaturer l'hémoglobine au moyen d'un changement conformationnel qui cause l'exposition d'un site de liaison de zinc à affinité élevée,
- capter les molécules d'hémoglobine en utilisant des molécules de zinc couplées à un milieu formant support, et
- déterminer la quantité d'hémoglobine glyquée liée par les molécules de zinc couplées.

2. Une méthode d'analyse telle que revendiquée dans la revendication 1 dans laquelle une dénaturation est causée par des détersifs.

3. Une méthode d'analyse telle que revendiquée dans la revendication 2 dans laquelle les détersifs sont un ou plusieurs détersifs parmi les détersifs sélectionnés dans le groupe consistant en cholate, N-octyl-glucoside, N-octyl-thioglucoside, triton X, zwittergent 3-14 et sels biliaires.

4. Une méthode d'analyse telle que revendiquée dans n'importe laquelle des revendications 1 à 3 dans laquelle la capture de l'hémoglobine par les molécules de zinc couplées est effectuée en la présence des détersifs de dénaturation.

5. Une méthode d'analyse telle que revendiquée dans n'importe laquelle des revendications 1 à 3 dans laquelle la molécule de zinc est couplée à une microparticule.

6. Une méthode d'analyse telle que revendiquée dans la revendication 5 dans laquelle la microparticule est une bille sélectionnée dans le groupe consistant en une bille de latex, une bille de polystyrène, une bille de silice, une bille de sépharose de chélation ou une bille magnétique.

7. Une méthode d'analyse telle que revendiquée dans la revendication 5 ou la revendication 6 dans laquelle le zinc couplé à la microparticule est fourni par du chlorure de zinc.

8. Une méthode d'analyse telle que revendiquée dans la revendication 5 dans laquelle la microparticule est du dextran ou du glycol polyéthylénique (PEG).

9. Une méthode d'analyse telle que revendiquée dans n'importe quelle revendication précédente dans laquelle un chélateur facilite le couplage des molécules de zinc et du milieu formant support.

10. Une méthode d'analyse telle que revendiquée dans la revendication 9 dans laquelle le chélateur est un agent chélateur de liaison de zinc.

11. Une méthode d'analyse telle que revendiquée dans la revendication 9 dans laquelle le chélateur est sélectionné dans le groupe consistant en acide éthylènediamine tétracétique (EDTA), diéthylènetriamine pentacétique de calcium trisodique (DTPA), cyclohexanediaminetétracétate de calcium disodium (CDTA), calcium édétate sodique, DMSA, DMPS, éthylènediamine (EDA), diéthylènetriamine (DETA) et éthylèneamines aminoéthyléthanolamines (AEEA).

12. Une méthode d'analyse telle que revendiquée dans n'importe quelle revendication précédente dans laquelle l'échantillon contenant l'hémoglobine est du sang total, des globules rouges, des hémolysats de sang ou des extraits de sang.

13. Une méthode d'analyse telle que revendiquée dans la revendication 12 dans laquelle l'échantillon contenant l'hémoglobine est des cellules de sang total qui sont lysées préalablement à la dénaturation.

14. Une méthode d'analyse telle que revendiquée dans la revendication 12 dans laquelle les globules rouges sont lysés en utilisant les détersifs qui causent également la dénaturation de l'hémoglobine.

15. Une méthode d'analyse telle que revendiquée dans la revendication 12 dans laquelle les globules rouges sont lysés en utilisant de la chaleur.

16. Une méthode d'analyse destinée à déterminer le niveau d'hémoglobine glyquée au sein d'un échantillon, la méthode incluant les étapes consistant à :
- extraire l'hémoglobine d'un échantillon de sang et dénaturer en partie l'hémoglobine présente,
- exposer l'hémoglobine à des microparticules revêtues de molécules de zinc afin de permettre une liaison de l'hémoglobine et de l'hémoglobine glyquée aux microparticules, et
- déterminer la quantité d'HbA1c liée aux microparticules.

17. La méthode d'analyse de la revendication 16, dans laquelle l'hémoglobine est extraite de l'échantillon par hémolyse de l'échantillon pour libérer l'hémoglobine liée aux cellules.

18. La méthode d'analyse de la revendication 16, dans laquelle la dénaturation partielle de l'hémoglobine au cours de l'étape de traitement est effectuée par des détersifs.

19. La méthode d'analyse de la revendication 18, dans laquelle les détersifs sont un ou plusieurs détersifs parmi les détersifs sélectionnés dans le groupe consistant en cholate, N-octyl-glucoside, N-octyl-thioglucoside, triton X, zwittergent 3-14 et sels biliaires.

20. La méthode d'analyse de la revendication 16, dans laquelle l'étape consistant à exposer l'hémoglobine aux microparticules revêtues de zinc est effectuée à un pH d'environ 7.

21. La méthode d'analyse de la revendication 16, dans laquelle la détermination de la quantité d'HbA1c présente est effectuée en mettant en contact l'HbA1c avec des molécules génératrices de signal qui se lient de façon sélective à l'HbA1c et de ce fait la marquent et la rendent détectable.

22. Une méthode d'analyse destinée au diagnostic in vitro et à la surveillance du diabète sucré grâce à l'évaluation de niveaux d'hémoglobine glyquée HbA1c, la méthode incluant les étapes consistant à :
- hémolyser un échantillon de sang pour libérer l'hémoglobine liée aux cellules et dénaturer en partie l'hémoglobine,
- capter l'hémoglobine glyquée sur des microparticules revêtues de zinc,
- exposer l'hémoglobine glyquée captée sur les microparticules à des molécules génératrices de signal à même de se lier à l'hémoglobine glyquée,
- retirer des molécules génératrices de signal qui ne sont pas liées à l'hémoglobine glyquée, et
- quantifier la quantité de molécules génératrices de signal liées pour déterminer le niveau de pourcentage d'HbA1c présent dans l'échantillon.

23. La méthode d'analyse de la revendication 22, dans laquelle le niveau de pourcentage d'HbA1c présent dans l'échantillon est déterminé grâce aux étapes supplémentaires consistant à :
- capter les microparticules sur un milieu de base adéquat,
- nettoyer les microparticules pour retirer de la matière non liée,
- marquer l'HbA1c d'une molécule génératrice de signal qui est un anticorps monoclonal spécifique pour l'HbA1c, l'anticorps monoclonal ayant une marque conjuguée à celui-ci, et
- déterminer le pourcentage d'HbA1c présent dans l'échantillon.

24. Une méthode pour isoler une hémoglobine d'un échantillon biologique, la méthode incluant les étapes consistant à :
- dénaturer en partie l'hémoglobine présente dans l'échantillon,
- capter l'hémoglobine de l'échantillon sur des microparticules qui sont revêtues de zinc, et
- éluer l'hémoglobine liée à la microparticule de la microparticule revêtue de zinc.

25. La méthode de la revendication 24, dans laquelle l'échantillon biologique est du sang.
